# EUROPEAN PATENT SPECIFICATION

(11) **EP 4 657 070 B1**
(45) Date of publication and mention of the grant of the patent: **28.10.2026**
(21) Application number: 25174974.3
(22) Date of filing: 08.05.2025
(51) Int. Cl.: G16B 40/10, G01N 33/68

(54) **METHODS FOR MS2 QUANTITATION OF ISOBARIC LABELED COMPOUNDS**
VERFAHREN ZUR MS2-QUANTIFIZIERUNG ISOBAR MARKIERTER VERBINDUNGEN
PROCÉDÉS DE QUANTIFICATION MS2 DE COMPOSÉS MARQUÉS ISOBARIQUES

(30) Priority: 29.05.2024 US 202463652766 P
(43) Date of publication of application: 03.12.2025
(73) Proprietor: Thermo Finnigan LLC, San Jose, CA 95134 (US)
(72) Inventor: REMES, Philip M., Livermore (US); MCALISTER, Graeme, San Jose (US)
(74) Representative: Boult Wade Tennant LLP

(56) References cited:
- MADERN MORITZ ET AL: "A Causal Model of Ion Interference Enables Assessment and Correction of Ratio Compression in Multiplex Proteomics", MOLECULAR & CELLULAR PROTEOMICS, vol. 23, no. 1, 12 December 2023 (2023-12-12), US, pages 100694:1 - 100694:2, XP093322857, ISSN: 1535-9476, DOI: 10.1016/j.mcpro.2023.100694
- MIKHAIL M. SAVITSKI ET AL: "Measuring and Managing Ratio Compression for Accurate iTRAQ/TMT Quantification", JOURNAL OF PROTEOME RESEARCH, vol. 12, no. 8, 2 July 2013 (2013-07-02), pages 3586 - 3598, XP055595390, ISSN: 1535-3893, DOI: 10.1021/pr400098r
- IWASAKI MIO ET AL: "Removal of Interference MS/MS Spectra for Accurate Quantification in Isobaric Tag-Based Proteomics", JOURNAL OF PROTEOME RESEARCH, vol. 18, no. 6, 30 April 2019 (2019-04-30), pages 2535 - 2544, XP093322608, ISSN: 1535-3893, DOI: 10.1021/acs.jproteome.9b00078
- PAULO JOAO A ET AL: "A Triple Knockout (TKO) Proteomics Standard for Diagnosing Ion Interference in Isobaric Labeling Experiments", JOURNAL OF THE AMERICAN SOCIETY FOR MASS SPECTROMETRY, ELSEVIER SCIENCE INC, US, vol. 27, no. 10, 11 July 2016 (2016-07-11), pages 1620 - 1625, XP036273617, ISSN: 1044-0305, [retrieved on 20160711], DOI: 10.1007/S13361-016-1434-9
- NISHANT PAPPIREDDI ET AL: "A Review on Quantitative Multiplexed Proteomics", CHEMBIOCHEM, vol. 20, no. 10, 18 April 2019 (2019-04-18), Hoboken, USA, pages 1210 - 1224, XP055678910, ISSN: 1439-4227, DOI: 10.1002/cbic.201800650
- TIAN XIAOBO ET AL: "Chemical isotope labeling for quantitative proteomics", MASS SPECTROMETRY REVIEWS., 6 June 2021 (2021-06-06), US, pages 1 - 31, XP055951707, ISSN: 0277-7037, DOI: 10.1002/mas.21709

## Description

### TECHNICAL FIELD

The present disclosure generally relates generally to the field of mass spectrometry. More particularly, the present disclosure relates to quantitative analyses of analyte compounds that are chemically labeled with one or more isobaric mass tags, each isobaric mass tag comprising a cleavable mass reporter portion and a mass normalizer portion detachably bonded to the cleavable mass reporter portion.

### BACKGROUND

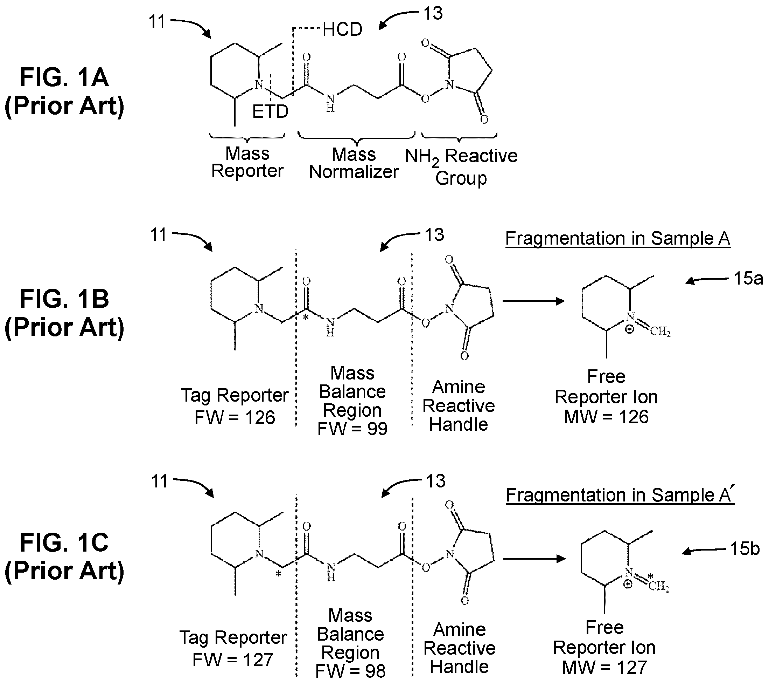

Multiplexed quantitation using Tandem Mass Tags (TMT), which is described, for example in Werner, T.; Becher, I.; Sweetman, G.; Doce, C.; Savitski, M. M.; Bantscheff, M. High-Resolution Enabled TMT 8-Plexing. Anal. Chem. 2012, 84 (16), 7188-7194, is an important technique in quantitative mass spectrometry (MS) proteomics that can increase sample throughput by at least an order of magnitude. An isobaric tag is a type of chemical label that can be used in a multiplexed analysis of peptides. The isobaric tags are fashioned so as to covalently attach to an analyte to form a tag-labeled analyte. Some examples of commercially available isobaric tags are TMT (Tandem Mass Tag Reagents, Thermo Scientific, Pierce Protein Research Product) and iTRAQ (Isobaric Tagging Relative Absolute Quantitation, SCIEX). During an MS/MS measurement, a "reporter ion" may be cleaved from an isobaric tagged peptide by various fragmentation methods. Each reporter ion, with its unique mass-to-charge *(m*/*z)* ratio, may then be detected, whereby the reporter ion's peak intensity/height/counts may be measured. A set of isobaric tags can be provided according to two or more types, where each type has a respective reporter ion portion **11** (FIGS. 1A-1C) where the reporter ion portion has a unique mass within the set. The area under a reporter-ion mass spectral peak (or the height of the peak) may afford a quantitative measurement of a tagged peptide as the peak area or intensity of the reporter ion peak may be proportional to its relative abundance.

To perform peptide quantitation in a multiplexing manner, a series of isobaric tag molecules may be chemically synthesized that contain various combinations of heavy isotopic atoms (for example, ²H, ¹³C, ¹⁵N, and ¹⁸O). Although the various reporter portions **11** may have different masses, the isobaric tags include compensatory mass-normalizing portions **13,** such that the total mass for each type of tag in the set is essentially the same (FIGS. 1A-1C), except for slight mass differences due to changes in mass defect. To compile a set of multiplexed isobaric tags from a base tag chemical structure, heavier isotopic atoms may be differentially substituted for their respective naturally occurring most abundant isotopic atoms (¹H, ¹²C, ¹⁴N, and ¹⁶O) in a balanced way between tag reporter moieties and mass balance regions of the tags. Heavier isotopic atoms are distributed to different parts of the same molecule such that each different tag reagent has the same nominal mass. Thus, all tagged molecules of a single peptide will have identical mass and thus may be simultaneously isolated within a mass spectrometer. FIG. 1A shows an example of a typical "base" TMT tag and illustrates how different MS fragmentation methods - in the example, electron transfer dissociation (ETD) and higher-energy dissociation (HCD) - may cause bond cleavage at different portions of a tag and thereby yield different types of reporter tag fragments.

Tag reporter ions may either have differing nominal masses or they may have a mixture of differing nominal masses and differing fractional masses (due to a mass defect effect). Accordingly, a series of samples (e.g., peptide mixtures) may be labelled with unique TMT tags using a series of multiplexed isobaric labels, and then the samples may be pooled and quantitatively analyzed concurrently. Fragmentation and detection of reporter-ion portions may then be used to identify and distinguish the provenance of otherwise compositionally identical peptides. This type of analysis is often used to detect the effects of different treatment procedures on otherwise identical samples that are labeled with different isobaric mass tags having different reporter ions.

For example, FIGS. 1B and 1C illustrate the generation of two different reporter ions from the two isobaric MS tag components of a two-plex isobaric MS tag kit sold by Thermo Fisher Scientific (Waltham, Massachusetts, USA). In practice, a first sample or sample portion (referred to here as Sample A) is labeled with the mass tag shown in FIG. 1B and a second sample or sample portion (referred to here as Sample A') is labeled with the mass tag shown in FIG. 1C. The molecular weights of both whole tags in the two-plex kit are the same (isobaric), but upon MS/MS fragmentation, a labeled peptide of Sample A would yield a tag reporter-ion fragment **15a** having a nominal m/z of 126 Da and the same peptide originating from Sample A' would yield a tag reporter-ion fragment **15b** having a nominal *m*/*z* of 127 Da. These *m*/*z* values are about one whole dalton apart, and are therefore easily baseline resolvable in any commercial tandem mass spectrometer. Since the differently labeled ions of the single common analyte comprise identical masses, they may be advantageously co-isolated in preparation for fragmentation. However, the various fragment ions that are generated by MS/MS fragmentation will comprise various different m/z values, depending on their tagging. Ideally, the relative abundances of the various fragment ions then correspond to the relative abundances of the protein or peptide analyte between the different samples (in this example, between Sample A and Sample A'). These relative abundances may be measured by the ratios of the detected reporter ions.

Although TMT, and its relative iTRAQ, are in principle powerful, the fact that every peptide in a sample fragments to form the same reporter ions has proved to be an encumbrance, leading to so-called "ratio distortion" and inaccurate measurements (Christoforou, A.; Lilley, K. S. Taming the Isobaric Tagging Elephant in the Room in Quantitative Proteomics. Nat Methods 2011, 8 (11), 911-913. Ratio distortion is a well-known problem that occurs frequently when using isobaric tags in mass spectrometry analysis. The problem arises when targeted precursor analyte ions are co-isolated and co-fragmented with other ions that are not of interest but that are also conjugated to isobaric tags. Both co-fragmented target and interfering ions produce the same reporter ions. As such, the "true" reporter ion ratios of targeted precursors can be obfuscated by the contribution of reporter ions derived from interfering ions. Depending upon sample complexity, and experimental conditions, ratio distortion problems can be quite severe. In experiments for which ratio distortion is severe, the median reporter-ion ratio (for example, when comparing tryptic peptide ratios for proteins from a "normal" cell to those of a diseased cell) will likely most often be 1:1 or "un-changed". This type of ratio distortion caused by interfering ions causes "ratio compression", which in-turn leads to an underestimation of statistically significant reporter-ion ratios. In other cases, the interfering ions may distort the ratios away from 1:1, in which case the observed data would falsely indicate that the protein abundances are different between the samples when in-fact the abundances are the same. In other cases, the interfering ion signals are by-products of the ionization and ion-injection process and are not simply co-eluting tryptic peptides.

Madern and coworkers addressed the ratio compression problem in multiplex proteomics, wherein an artificial two-proteome dataset (yeast and human) is used for exploring ion interference and for building a model for calculating corrected reporter-ion ratios (Madern M. et al., "A Causal Model of Ion Interference Enables Assessment and Correction of Ratio Compression in Multiplex Proteomics",Mol. Cell. Proteomics 2023, 23(1), 100694). Savitski and coworkers addressed the ratio compression problem in multiplex proteomics using an algorithm based on measures of spectrum purity (Savitski M. M. et al., "Measuring and Managing Ratio Compression for Accurate iTRAQ/TMT Quantification", J. Proteome Res. 2013, 12(8), 3586-3598). Iwasaki and coworkers addressed the problem of interferences in tandem mass spectrometry quantification in isobaric tag-based proteomics, wherein correcting ratio distortion is performed by estimating the extent of interference or by detecting and discarding MS2 spectra that are produced from multiple precursor ions (Iwasaki M. et al., "Removal of Interference MS/MS Spectra for Accurate Quantification in Isobaric Tag-Based Proteomics", J. Proteome Res. 2019, 18(6), 2535-2544). Joao and coworkers addressed the problem of ion interference in isobaric labelling experiments by using a multiplexed tandem mass tag-based standard, termed the triple knockout (TKO) (Joao J. A. et al., "A Triple Knockout (TKO) Proteomics Standard for Diagnosing Ion Interference in Isobaric Labeling Experiments", J. Am. Soc. Mass Spectrom. 2016, 27(10), 1620-1625). The problem of ratio distortion (also called ratio compression) in tandem mass spectrometry using isobaric tags (also called tandem mass tags or TMTs) is a know problem (see the following review articles: Pappiredi N. et al., "A Review on Quantitative Multiplexed Proteomics", ChemBioChem 2019, 20(10), 1210-1224; Tian X. et al., "Chemical isotope labeling for quantitative proteomics", Mass Spectrom Rev. 2021, 1-31).

Ting and coworkers showed that the inclusion of an additional round of MS selectivity (e.g., MS3 analysis), may obviate the signal contribution from interfering ions (Ting, L.; Rad, R.; Gygi, S. P.; Haas, W. MS3 Eliminates Ratio Distortion in Isobaric Multiplexed Quantitative Proteomics. Nat Methods 2011, 8 (11), 937-940.). Although MS3 analysis can effectively reduce ratio distortion, it is much slower than MS2 analysis, and leads to a loss in sample coverage. Accordingly, the inventors have recognized that there is a need in the art for methods for quantitative MS2 analysis of isobaric mass-tag-labeled samples and other similarly multiply labeled samples in the absence of significant ratio distortion.

### SUMMARY

In this disclosure, the inventors describe a computational technique to achieve high throughput analysis of tandem mass tag (TMT) labeled analytes and other isobarically labeled analytes using MS2 mass spectral analyses. The basis of the method is that the mass spectrometric signal in the reporter-ion m/z channels may, in many cases, be decomposed into multiple components, comprising a first signal, the magnitude of which is strongly correlated with the variation in abundance of the precursor peptide of interest, and a second signal that arises from chemical constituents whose abundances are not strongly correlated with the abundance of the precursor of interest. If there are m/z channels in the full MS2 spectrum that are known to be correlated exclusively with the precursor of interest, and other m/z channels that are known to not be strongly correlated with the precursor of interest, then it is possible to estimate the amount of reporter ion signal that is due to the precursor of interest, thereby improving quantitative accuracy.

By employing the novel techniques described herein, it is possible to at least partially compensate for the so-called ratio distortion that is inherent to multiplexed isobaric labeling techniques, such as TMT and iTRAQ. The methods described herein are, in principal, amenable to quantitative analysis of mass spectra of multiplexed isobaric-labeled analytes obtained by all types of mass spectrometers, but especially mass spectrometers capable of performing full-scan MS/MS (i.e., "MS2) analyses. These techniques can be applied whenever a plurality of mass spectra of a compound are acquired during a time in which an experimental or instrumental parameter is varied. Most commonly, the varied parameter is related to the liquid chromatography (LC) elution profile for the compound. However, in principal, other experimental or instrumental operational parameters could be varied to find a separation between the mass spectrometric signal of the compound of interest and the signals of other components. The types of experimental or instrumental operational parameters that may be varied include, without limitation, ion mobility drift time, compensation voltage of a Field Asymmetric Ion Mobility Separation (FAIMS) analysis, and MS2 isolation window placement and width, in terms of *m*/*z* (for isolation by either an ion trap or a quadrupole mass filter). Thus, the novel methods of the present teachings could be applied to targeted MS2 experiments, also known as parallel reaction monitoring (PRM), data dependent acquisition (DDA), or data independent acquisition (DIA) experiments. An advantageous aspect of the methods of the present teachings is that MS3 mass spectroscopic analysis can be avoided in many cases, thereby allowing for maximum instrument speed and a maximum number of compounds that can be analyzed in an experiment. Nonetheless, even MS3 scans can sometimes suffer from ratio-compression, and the present teachings described herein are also applicable to MS3 acquisitions as well.

According to a first aspect of the present teachings, there is provided a method for correcting abundance ratios between pairs of reporter ions detected during tandem mass analysis (MS2) of a sample that comprises a plurality of peptides that are labeled with a plurality of different isobaric tags, each isobaric tag comprising a respective reporter-ion moiety that comprises a respective predetermined reporter-ion formula weight and a respective mass-balancing moiety that comprises a respective predetermined mass-balancing formula weight, whereby all isobaric labels comprise identical masses, the method comprising:
during a time period, measuring, for each reporter-ion moiety liberated by the tandem mass analysis, the variation, with time, of a mass spectrometric signal derived from said respective reporter-ion moiety;
identifying a first set of reporter-ion moieties for which the respective mass spectrometric signal is positively correlated, with an above-threshold correlation coefficient, with the time variation, during the time period, of one or more other mass spectrometric signals, parameters or variables that pertain to the detection of a peptide of interest and identifying a second set of reporter-ion moieties for which the respective mass spectrometric signal is not positively correlated with the one or more other mass spectral signals or variables;
based on the correlations, for each identified reporter-ion moiety of the first set, decomposing the respective measured mass spectrometric signal into a first portion that is attributable to the peptide of interest, and a second portion that is not attributable to the peptide of interest;
for each identified reporter-ion moiety, setting a respective adjusted mass spectrometric signal as being the respective portion of the mass spectrometric signal that is attributable to the peptide of interest; and
calculating corrected reporter-ion ratios based on the adjusted mass spectrometric signals.

In accordance with some embodiments, the decomposing of each measured mass spectrometric signal into a respective first portion that is attributable to the peptide, and a second portion that is not attributable to the peptide comprises calculating the first and second portions using a non-negative least squares analysis.

In accordance with some embodiments, the time variation of the one or more other mass spectrometric signals, parameters or variables that pertain to the detection of one or more peptides of interest are determined from tabulated values of peptide elution times and/or elution profiles. In accordance with some other embodiments, the time variation of the one or more other mass spectrometric signals, parameters or variables that pertain to the detection of one or more peptides of interest is determined by measuring the variation of one or more other mass spectrometric signals or variables during the time period.

In accordance with some embodiments, the one or more other mass spectrometric signals or variables that are measured during the time period comprise mass spectra of characteristic peptide fragment ions.

In accordance with some embodiments, the beginning of the time period is triggered by a time delay relative to the beginning of an experiment. In accordance with some other embodiments, the tandem mass analysis of the sample comprises a data-dependent analysis (DDA) experiment, wherein the beginning of the time period is triggered by detection of an *m*/*z* value, within an MS1 mass spectrum, that corresponds to a peptide of interest. In accordance with some other embodiments, the beginning of the time period is triggered by the prior steps of: (a) detection of a first *m*/*z* value, within an MS1 mass spectrum, that corresponds to a potential peptide of interest; (b) automatic consultation of a mass spectral library to determine a second *m*/*z* value to be searched for within a subsequent MS2 mass spectrum; (c) generation of fragment ions by fragmentation of ions comprising the first *m*/*z* value; and (d) detection of the second *m*/*z* value within an MS2 spectrum of the fragment ions. According to yet other embodiments, the beginning of the time period is triggered by elapse of an ion mobility drift time subsequent to the introduction of a stream of peptide ions labeled with the plurality of different isobaric tags into an ion mobility separation device that receives the ions from an ion source and that delivers ion mobility separated ions to a mass spectrometer that performs the tandem mass analysis. According to yet other embodiments, the tandem mass analysis of the sample comprises a data-independent analysis (DIA) experiment, wherein the time period is one of a plurality of scheduled overlapping time periods.

According to a second aspect of the present teachings, A mass spectrometer system comprises:
an ion source that generates first-generation ions from a sample;
an ion trap or reaction cell configured to receive first-generation ions from the ion source;
a mass analyzer configured to receive a portion of the first-generation ions or fragment ions generated by fragmentation of a portion of the first-generation ions from the ion trap or reaction cell;
one or more electrical power supplies electrically coupled to the ion source, the ion trap or reaction cell and the mass analyzer; and
a computer processor electrically coupled to the ion source, the ion trap or reaction cell and the mass analyzer and comprising tangibly-embodied non-transitory computer instructions that are operable to cause the one or more power supplies to provide voltages or electrical signals to the ion source, the ion trap or reaction cell and the mass analyzer that cause the mass spectrometer system to execute the steps of:
   during a time period, measuring, for each reporter-ion moiety liberated by the tandem mass analysis, the variation, with time, of a mass spectrometric signal derived from said respective reporter-ion moiety;
   identifying a first set of reporter-ion moieties for which the respective mass spectrometric signal is positively correlated, with an above-threshold correlation coefficient, with the time variation of one or more other mass spectrometric signals, parameters or variables that pertain to the detection of one or more peptides of interest and identifying a second set of reporter-ion moieties for which the respective mass spectrometric signal is not positively correlated with the one or more other mass spectral signals or variables;
   for each identified reporter-ion moiety, decomposing the respective measured mass spectrometric signal into a first portion that is attributable to the peptide, and a second portion that is not attributable to the peptide;
   for each identified reporter-ion moiety, setting a respective adjusted mass spectrometric signal as being the respective portion of the mass spectrometric signal that is attributable to the peptide; and
   calculating corrected reporter-ion ratios based on the adjusted mass spectrometric signals.

### BRIEF DESCRIPTION OF THE DRAWINGS

The above noted and various other aspects of the present invention will become apparent from the following description which is given by way of example only and with reference to the accompanying drawings, not necessarily drawn to scale, in which:
FIG. 1A is a representation of a chemical structure of an isobaric mass tag comprising a cleavable mass reporter portion, a mass normalizer portion, and an NH₂ reactive group region adapted for adduction onto a peptide structure;
FIG. 1B is a depiction of the chemical structure of a first isobaric mass tag, of the general type shown in FIG. 1A, that has a reporter portion and mass balance portion that, together, have a formula weight of 125 together with a depiction of the reporter ion portion, having a formula weight of 126 and a charge state of +1, that may be generated by fragmentation of the isobaric mass tag within a mass spectrometer;
FIG. 1C is a depiction of the chemical structure of a second isobaric mass tag, of the general type shown in FIG. 1A, that has a reporter portion and mass balance portion that, together, have a formula weight of 125 together with a depiction of the reporter ion portion, having a formula weight of 127 and a charge state of +1, that may be generated by fragmentation of the isobaric mass tag within a mass spectrometer;

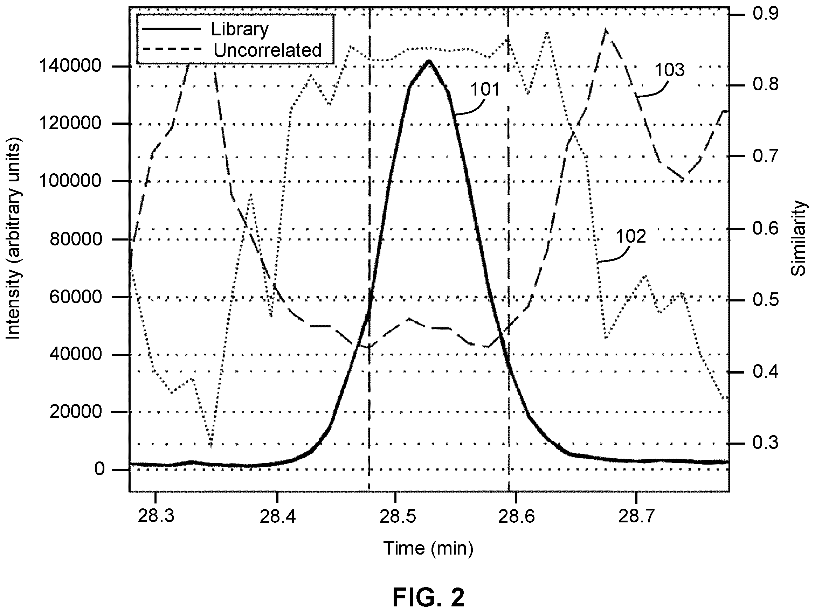

FIG. 2 is a set of graphs depicting chromatographic and mass spectrometric analysis of the peptide E[TMTLabel]YFEAANK[TMTLabel] showing: a first trace (solid line) depicting the composite signal of *m*/*z* channels (for instance, *y*-ions) that are known to correspond to the elution profile of a peptide of interest, a second trace (dashed line) depicting the signal in *m*/*z* channels that have low correlation (e.g., negative correlation) to the signal of the first trace, and a third trace (dotted line) showing the normalized dot product between the first trace and a hypothetical elution profile of the peptide of interest as determined from a stored mass spectral library;

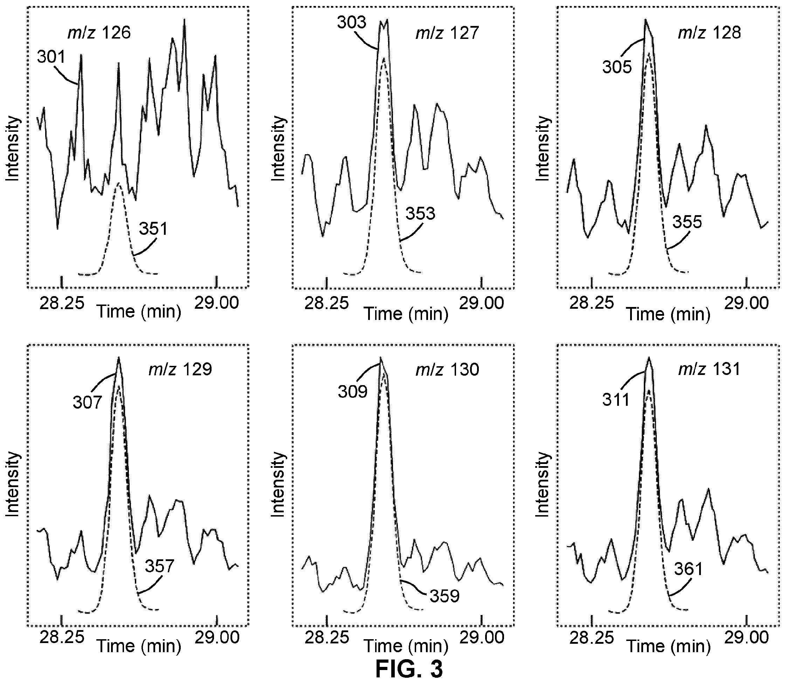

FIG. 3 is a set of traces of raw measured mass spectral signals at the *m*/*z* positions of various reporter ions (solid lines), as measured in a sample comprising a mixture of isobarically labeled peptides, plotted in comparison to the corresponding respective calculated contributions to each raw signal (dashed lines), as calculated by a method in accordance with the present invention;

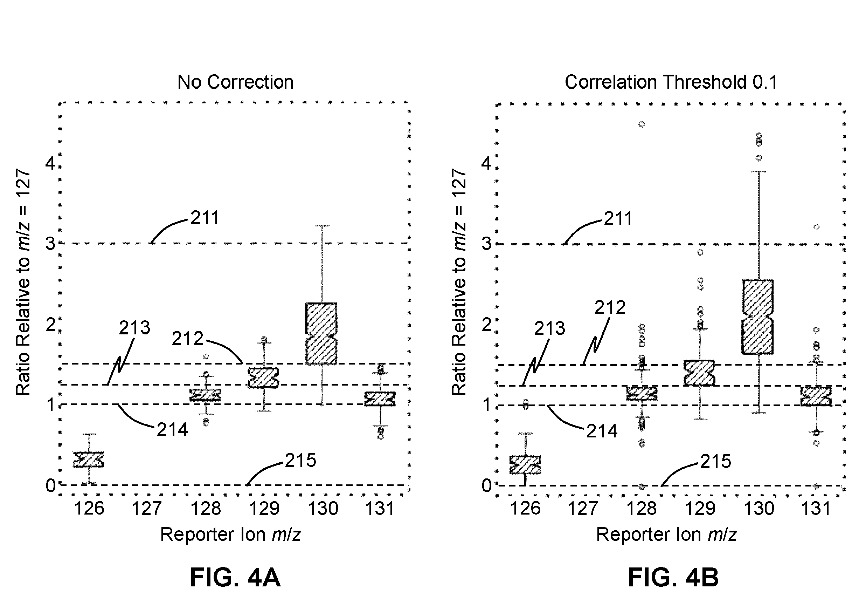

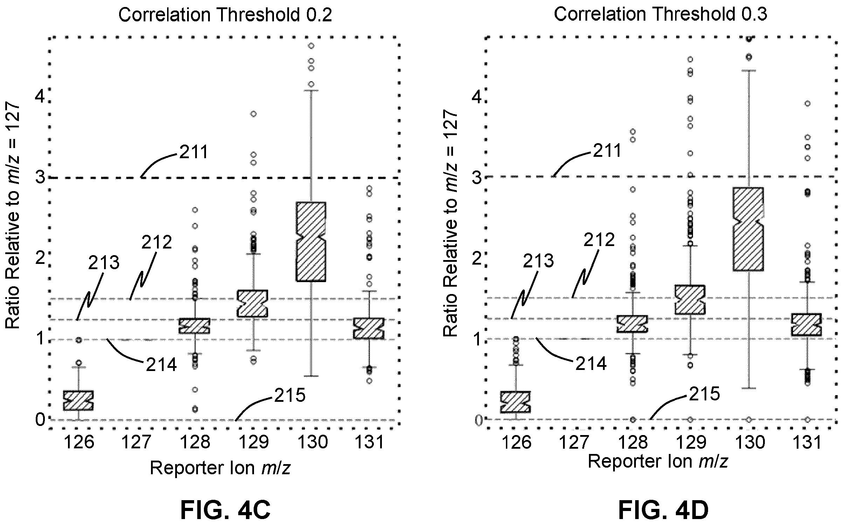

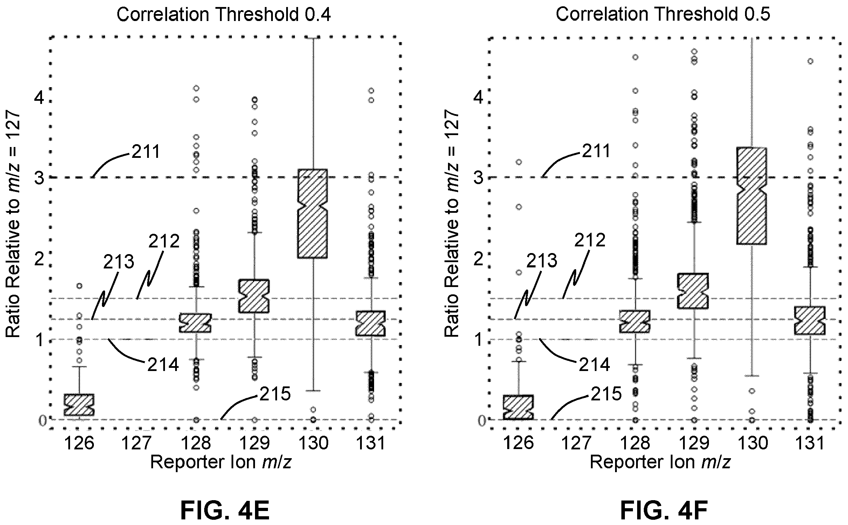

FIGS. 4A-4D are a group of plots of observed distributions of reporter ion ratios at various levels of threshold correlation level, used to designate m/z channels as uncorrelated from the precursor of interest;

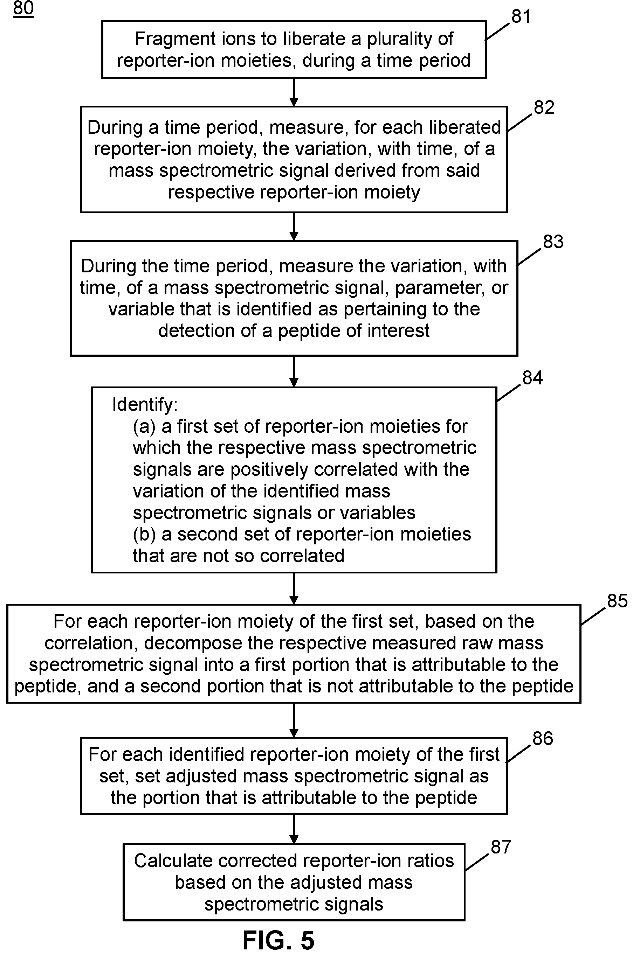

FIG. 5 is a flow diagram of an exemplary method in accordance with the present teachings; and

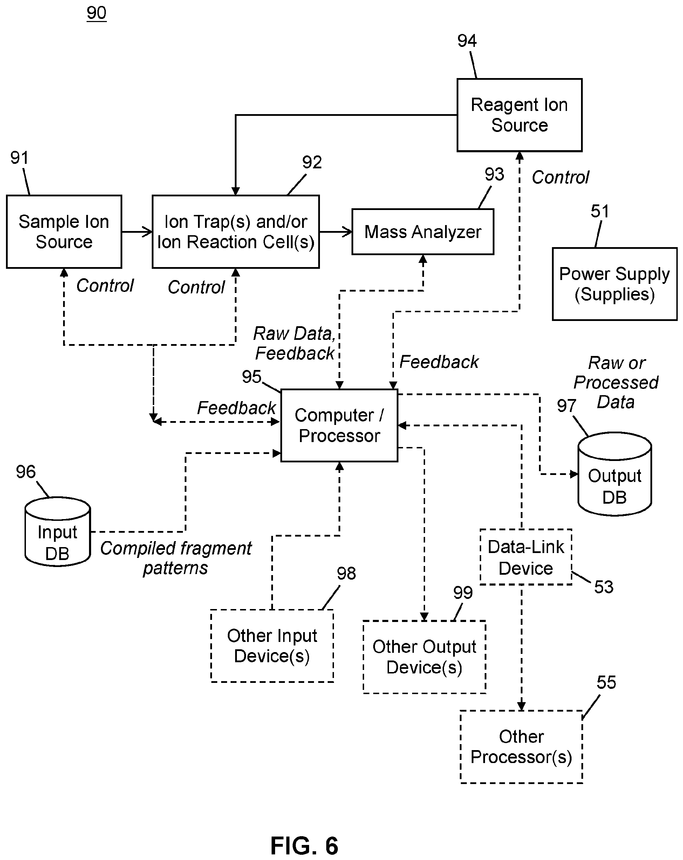

FIG. 6 is a schematic illustration of a mass spectrometry system in accordance with the present teachings.

### DETAILED DESCRIPTION

The following description is presented to enable any person skilled in the art to make and use the invention and is provided in the context of a particular application and its requirements. Various modifications to the described embodiments will be readily apparent to those skilled in the art and the generic principles herein may be applied to other embodiments. Thus, the present invention is not intended to be limited to the embodiments and examples shown but is to be accorded the widest possible scope in accordance with the features and principles shown and described. To fully appreciate the features of the present invention in greater detail, please refer to FIGS. 1A-1C, 2, 3, 4A-4F, 5 and 6 in conjunction with the following description.

In the description of the invention herein, it is understood that a word appearing in the singular encompasses its plural counterpart, and a word appearing in the plural encompasses its singular counterpart, unless implicitly or explicitly understood or stated otherwise. Furthermore, it is understood that, for any given component or embodiment described herein, any of the possible candidates or alternatives listed for that component may generally be used individually or in combination with one another, unless implicitly or explicitly understood or stated otherwise. Moreover, it is to be appreciated that the figures, as shown herein, are not necessarily drawn to scale, wherein some of the elements may be drawn merely for clarity of the invention. Also, reference numerals may be repeated among the various figures to show corresponding or analogous elements. Additionally, it will be understood that any list of candidates or alternatives is merely illustrative, not limiting, unless implicitly or explicitly understood or stated otherwise. Unless otherwise explicitly stated, the phrase "A or B" is understood to be "true" when: only "A" is true, when only "B" is true, as well as when both "A" and "B" are true.

The inventive techniques are here described in terms of a representative example in which Liquid-Chromatography / Mass-Spectrometry (LCMS) analysis of a set of multiplexed compounds labeled with isobaric reagents is performed. Upon introduction of a liquid chromatography (LC) eluent fraction into a mass spectrometer, the compounds within each eluent fraction are ionized and ions having m/z values within selected ranges are fragmented. This fragmentation releases the various reporter ions, each reporter ion having a respective formula weight that corresponds to a respective m/z channel (i.e., a specific reporter-ion "channel").

According to one method embodiment in accordance with the present teachings, a respective extracted-ion chromatogram (XIC) may be determined for each reporter ion channel subsequent to mass spectrometric data collection Each such XIC is a record of the time variation of the detected number of reporter ions within the *m*/*z* range of the channel. As such, each XIC is essentially an elution profile of one or more compounds - some of which may not be analytes of interest as a result of co-elution - to which the reporter ions of the channel were bonded prior to fragmentation. The inventors have determined that, given a known or predicted chromatographic elution profile of an analyte of interest, such as a protein or peptide analyte, some XIC profiles are highly positively correlated with the known/predicted analyte elution profile while other XIC profiles are uncorrelated (correlation coefficient near zero) or negatively correlated with the known or predicted elution profile. Each reporter-ion channel may then be categorized as either: (a) corresponding to a known fragment of the analyte, if there is an above-threshold calculated positive correlation between the XIC and the known/predicted elution profile; (b) having an XIC profile that is uncorrelated with the known analyte elution profile, if the calculated correlation coefficient is below the threshold; or (c) undetermined. After discarding the channels of category "(c)", the signals of channels of category "(a)" may be summed, timepoint-by-timepoint and the resulting sum subsequently normalized. Likewise, the channels of category "(b)" may be summed, timepoint-by-timepoint, and the resulting sum thereby normalized. Each set of summed points comprises a respective vector. These two vectors may then be used to decompose the signal from each reporter ion *m*/*z* channel into separate components (e.g., signal contributions) that result from: (i) elution of an analyte of interest and (ii), elution of interfering, co-eluting compounds that incorporate the same respective reporter ion. The observed ratios between the signals corresponding the various reporter ions may be then corrected by calculating the ratios using only the signal contributions that correlate with the elution of the analyte of interest.

### Experimental Procedure

An experiment was performed on a sample known as the "HYPER standard", in which yeast samples are labeled in different known proportions for each of 11 TMT channels and are mixed with a background of human digest. This sample is an excellent platform on which to test the performance of quantitative measurements, since the yeast compounds are mixed in known ratios and the human background causes significant ratio distortion using normal MS2 and normal summing of the areas of the reporter ion m/z channels. This is the same sample that was analyzed in Schweppe et al. (Schweppe, D. K.; Prasad, S.; Belford, M. W.; Navarrete-Perea, J.; Bailey, D. J.; Huguet, R.; Jedrychowski, M. P.; Rad, R.; McAlister, G.; Abbatiello, S. E.; Woulters, E. R.; Zabrouskov, V.; Dunyach, J.-J.; Paulo, J. A.; Gygi, S. P. Characterization and Optimization of Multiplexed Quantitative Analyses Using High-Field Asymmetric-Waveform Ion Mobility Mass Spectrometry. Anal. Chem. 2019, 91 (6), 4010-4016.), in which analysis was performed using a linear ion trap mass analyzer, such that only 6 channels could be mass resolved. The *m*/*z* 126 channel contains only a single isotopologue, while the channels 127, 128, 129, 130, and 131 each contain two isotopologues separated by a very small m/z difference, meaning that the average of the two channels will be measured in these cases. The sample was characterized with an initial discovery experiment to identify peptides of interest, and a parallel reaction monitoring (PRM) mass spectrometric assay was developed for each of 1000 of these peptides. During the PRM experiment, the instrument was directed to acquire a set of MS2 spectra to detect each respective peptide during a time period when the peptide is known to elute. The presence of the peptide of interest could be confirmed based on comparison to a spectral library.

More specifically, each such tandem MS2 spectral measurement comprises, during each time period when one of the peptides of interest is known to elute: (1) optionally, obtaining an initial mass spectrum (i.e., an MS1 spectrum) of ions to confirm the presence of ions (e.g., at an expected *m*/*z* value) of the expected peptide at the respective expected time period and recording a signal versus time that relates to the variation, during the respective expected time period, of an intensity of a mass spectrometric signal that pertains to the ions of the expected peptide; (2) isolating a subset of the ions that are introduced into the mass spectrometer, the subset comprising ions at the expected *m*/*z* value; (3) fragmenting the isolated ions by a fragmentation procedure that liberates ions of a plurality of reporter-ion species from the isolated ions, each reporter-ion species having a respective known reporter-ion m/z value; and (4) detecting and recording, versus time during each respective time period, raw mass spectrometric signals (i.e., MS2 spectra) that pertain to the variation of the intensity of each reporter ion species during the time period. The acquired raw reporter-ion signals may then be refined and corrected using the data analysis procedure described below.

### Data Analysis

FIG. 2 shows measured profiles of measured signal intensity of fragment ions within several *m*/*z* bins (i.e., restricted *m*/*z* ranges) during elution of the peptide E[TMTLabel]YFEAANK[TMTLabel]. In this figure, solid-line trace **101** shows the summed signal from several *m*/*z* bins that are *a priori* known to correspond to ions that are formed from the above-identified peptide. Solid-line trace **101** may therefore be taken as an elution profile of the peptide, itself. Dashed-line trace **103** corresponds to the signal from other *m*/*z* bins that have low correlation, and even negative correlation, to trace **101.** Trace **103** exhibits a much different time profile than the profile (trace **101)** that corresponds to the "known fragment" m/z channels and thus may be taken as a signal from one or more interfering co-eluting compounds of unknown identity. Dotted-line trace **102** is the normalized dot product between trace **101** to an elution profile calculated for the peptide from a stored spectral library. The similarity score between trace **101** and trace **102** is great enough to give a measure of confidence that this compound is actually present. All of the m/z channels in these MS2 spectra were analyzed to compute their correlation in time to the "known fragment" m/z channels (trace **101).** Trace **103** was then generated by pointwise summing of the intensities of those channels with correlation scores less than a threshold value.

The profile-versus-profile similarity determination procedures described above permit assignment of each reporter-ion m/z channel to one of the general categories: "Known fragment", "Uncorrelated", and "Other". After discarding the peaks in the "Other" category, the channels of the "Known Fragment" category may all be summed together and the resulting sum normalized. Likewise, the channels corresponding to the "Uncorrelated" category may be summed and normalized. The resulting two vectors are then used to decompose the signal from each individual reporter-ion channel into a respective "Known fragment" component and a resulting "Uncorrelated" component, using non-negative least squares analysis. This problem can be represented in the canonical *AX = B* form, or equivalently in Equation 1, $\left[K U\right] \left[\begin{matrix}{x}_{{k}_{126}} & … & {x}_{{k}_{131}} \\ {x}_{{u}_{126}} & … & {x}_{{u}_{131}}\end{matrix}\right] = \left[{R}_{126} … {R}_{131}\right]$ Eq.1 where the "A matrix" is composed of normalized column vectors, *K* and U, that are determined from the peptide fragment-ion signals that correspond, respectively, to the correlated and uncorrelated signals; the "B matrix" is composed of the various observed reporter-ion-channel signal vectors, *Rₙₙₙ*, *(nnn =* 126, 127, etc., where each value of *nnn* represents a respective *m*/*z*); and the *X* matrix contains the scalar decomposition values for the proportions of "Known" (subscripts, k) and Unknown (subscripts, u) components that contribute to the signal of each reporter-ion channel and that are to be solved for by the non-negative least squares procedure.

This is just one instance of how the problem of solving for "Known" and "Unknown" signal components could be setup. For example, it may be noted that an alternative way of determining the true elution profile of a precursor of interest is to use the normalized median of the fragments at each time point. This latter procedure gives a smoother and possibly more robust estimate of the elution profile and was described briefly by Searle et al. (Searle, B. C.; Pino, L. K.; Egertson, J. D.; Ting, Y. S.; Lawrence, R. T.; MacLean, B. X.; Villén, J.; MacCoss, M. J. Chromatogram Libraries Improve Peptide Detection and Quantification by Data Independent Acquisition Mass Spectrometry. Nat Commun 2018, 9 (1), 5128.) and, in more detail, by Remes et al. (Remes, P. M.; Yip, P.; MacCoss, M. J. Highly Multiplex Targeted Proteomics Enabled by Real-Time Chromatographic Alignment. Anal. Chem. 2020, 92 (17), 11809-11817.) A similar operation could be performed for the reporter-ion channels in the "Uncorrelated" category.

Alternatively, given a series of mass spectra, there are many known techniques for determining the number of components that are eluting, such as factor analysis (e.g., Knorr, F. J.; Futrell, J. H. Separation of Mass Spectra of Mixtures by Factor Analysis. Anal. Chem. 1979, 51 (8), 1236-1241.). These more sophisticated means could be used to determine a plurality of categories into which the reporter-ion channels may be assigned. For instance, analysis may determine that a specific number, *N*, of components other than the known compound are present, and these could be accounted for by *N* columns in the *A* matrix, instead of the single *K* column of Eq. 1, where each of the *N* columns contain a signal representative of a particular component.

Returning to the discussion of Eq. 1, once the scalar values *x_{kᵢ}* are determined, they are simply multiplied by the *K* column to yield the estimated amount of reporter ion signal in each channel that comes from the peptide of interest. This is what one might want to do in the case of highly multiplexed spectra where multiple peptides of interest are present. Generally, however, it is only necessary to: (a) determine at least one reporter-ion channel or signal that originates from or that exclusively represents the peptide of interest for the *K* column; and (b) find all the *m*/*z* channels or a signal representative of components other than peptide of interest in the *U* column, excluding from it any *m*/*z* channels of mixed parentage. The time behavior of these two signals allows for an estimation of the *x_{kᵢ}*. Although it is not necessary to separate signals from the "Uncorrelated" channels into the actual number of components that produced them, estimation errors will arise if *m*/*z* channels are included in the "Uncorrelated" category that actually have contribution from the peptide of interest.

### Results

The above-described data analysis procedure was carried out for the peptide E[TMTLabel]YFEAANK[TMTLabel], yielding the various traces shown in FIG. 3. The solid-line curves **301, 303, 305, 307, 309, 311** in FIG. 3 are the raw signal in each of the six reporter ion channels (i.e., reporter-ion channels *"m*/*z* 126", *"m*/*z* 127", *"m*/*z* 128", *"m*/*z 129", "m*/*z* 130" and *"m*/*z* 131", respectively), while the dashed traces **351, 353, 355, 357, 359, 361** represent the respective amount of reporter-ion signal that is calculated to result from elution of the peptide of interest. The *"m*/*z* 126" channel is unique, because it is known not to contain any contribution from the yeast peptide of interest, and so represents the background signal, possibly resulting from co-elution.

The *"m*/*z* 127" channel is the reference channel and thus serves as an internal standard for calibrating reporter-ion ratios relating to the other reporter-ion channels. Raw reporter-ion signal ratios, relative to the reference, were calculated by integrating the areas under the peaks of the solid-line traces **301, 305, 307, 309, 311** and dividing the resulting integrated area of each channel, other than the *"m*/*z* 127" channel, by the integrated area under the curve representing the *"m*/*z* 127 channel". Adjusted reporter-ion signal ratios, relative to the reference, were calculated by integrating the areas under the peaks of the dashed-line traces **351, 355, 357, 359, 361** and dividing the resulting adjusted integrated area of each channel, other than "the *m*/*z* 127" channel, by the adjusted integrated area under the curve **353** of the *"m*/*z* 127" channel. Note that, because the *m*/*z* 127 channel is the reference, its signal ratio is always equal to unity.

Table 1 shows the expected, raw and adjusted area ratios for each channel. For this one example, the table shows that the data analysis and adjustment methods,

**Table 1**

| Channel | Expected Ratio | Raw Ratio | Adjusted Ratio |
|---|---|---|---|
| *m*/*z* 126 | 0.00 | 0.22 | 0.14 |
| *m*/*z* 127 | 1.00 | 1.00 | 1.00 |
| *m*/*z* 128 | 1.25 | 1.19 | 1.25 |
| *m*/*z* 129 | 1.50 | 1.46 | 1.58 |
| *m*/*z* 130 | 3.00 | 2.32 | 2.72 |
| *m*/*z* 131 | 1.50 | 1.24 | 1.35 |

as described above, bring the adjusted channel ratios closer to their expected values in every case. The *"m*/*z* 130" channel has the greatest intensity, and ratio-compression distortion is usually prominently observed in this channel. The adjusted ratio is 2.72, compared to the raw ratio of 2.32, and the expected ratio of 3.0. The *"m*/*z* 131" channel is also interesting, because it contains two isotopologues, one of which has no relation to the yeast sample and only contributes human background. This channel is frequently distorted, although the adjustment reduces some of the distortion.

The above-described single example is presented in order to assist the reader in understanding the details of the inventors' novel method, but, to determine if the method has any practical merit, it is necessary to repeat the analysis using many samples. The inventors have performed such a study for 999 peptides, using the aforementioned one-hour PRM mass spectrometric assay with a specification that the total summed area should be at least 1000 units. Further, the entire data analysis procedure was repeated at various values of a correlation threshold that determines which *m*/*z* bin regions are included in the "Known" column, *K,* of Eq. 1.

The results of the measurements and data analyses of the 999 peptides are depicted, in FIGS. 4A-4F, as box and whisker plots that show the statistical distributions of calculated raw area ratios (FIG 4A) and calculated corrected area ratios (FIGS. 4B-4F), relative to the *"m*/*z* 127" channel, for each of the other reporter-ion channels. FIG. 4A shows the distribution of calculated reporter ion ratios using solely raw MS2 data without application of the above-described procedures for correction or ratio distortion. FIGS. 4B-4F show calculated distributions of the reporter ion ratios, with the corrections applied and with progressively increasing correlation coefficient threshold. The uncorrected condition (FIG. 4A) shows that, with the exception of the background "*m*/*z* 126" channel, all the calculated ratios are less than their expected values, these expected values being graphically depicted by the horizontal, dashed lines **211, 212, 213, 214,** and **215** which pertain respectively, to: *"m*/*z* 126", *"m*/*z* 127", *"m*/*z* 128", both of *"m*/*z* 129" and *"m*/*z* 131" and *"m*/*z* 130" (see Table 1 above).

The data representations presented in FIGS. 4A-4F show that, except for the background *"m*/*z* 126" channel, that is greater than the expected ratio of zero, and the "m/z 127" channel, which is defined as unity, all calculated reporter ion ratio values are less than expected, indicating the continued existence of some ratio distortion effects. As the correlation threshold is raised, the distribution of ratios and their mean values progressively move closer to the respective expected values. However, it is also apparent that the distributions progressively grow somewhat broader as the correlation threshold is increased, with a number of outliers (represented by circles) making their appearance. Nonetheless, the overall results indicate that the correction methods described herein are useful in reducing ratio distortion.

Although, in the above discussion, the ratio distortion correction method has been described in the context of a PRM experiment, there are many other ways it may be employed. For example, in a DDA experiment, one may specify that, upon initial detection of the *m*/*z* value of a potential precursor ion of interest in an MS1 scan and during time period of its elution, then a number, N, of MS2 mass spectral measurements should be acquired for the precursor ion for the purpose of measuring the signals of reporter ions that are liberated by the MS2 scans during the elution. In another embodiment this method could be combined with a real-time search of mass spectral library data, such as MS2 library spectra. In this latter case, if an MS2 scan produces a spectral match to a precursor of interest, as indicated by the library search, then that same precursor would be interrogated again using additional MS2 scans across the compounds LC elution for the purpose of measuring the signals of reporter ions that are liberated by the MS2 scans during the elution. If the initial MS2 measurement does not produce a spectral match to a library entry, then the precursor *m*/*z* value would be excluded from any further MS2 analysis for a set exclusion period.

A similar technique would be amenable to data independent analysis (DIA) experiments. The larger isolation widths that are typically utilized for DIA would make the determination of the components in the spectra more challenging than when such determinations are made for PRM experiments as described above. Nonetheless, the job of identifying components in DIA spectra is already performed by a number of different software packages with at least some measure of success, thereby facilitating its extension through the use of reporter-ion ratio correction techniques similar to those described herein. In the case of DIA experiments, a possible workflow would be to first identify all components in the spectra, possibly in a set of discovery runs on an aliquot of pooled samples, as is commonly done to build a chromatogram library. Then, in later runs, for each identified component of interest, the K column (Eq. 1) may be generated from one or more *m*/*z* bins that are known to have signal from that component, and *U* may be generated from all *m*/*z* channels that do not have signal from that component. The DIA case may suffer from accuracy problems, however, because of accumulated errors, the signal of interest being much smaller than other contributions, and the decreasing likelihood that a signal representing the component of interest can be resolved from the other components.

As mentioned above, whereas the above-described method for correcting reporter-ion ratio distortion utilizes differences in elution times to differentiate signals from the peptide of interest and other signals, various other means of separating ion species are known in the art and could be of utility. The only basic requirement is that the variation of a raw reporter-ion signal that is measured, during such ion separation, may be expressed in terms of a first component that varies in a correlated fashion with the variation of a signal that is attributable to a detection of or an otherwise-determined presence of a known chemical constituent (e.g., a peptide) and a second component whose variation has low or no correlation with the variation of the signal attributable to the known constituent. For example, a series of spectra taken as a function of ion mobility drift or trapping time may help to separate components, as could sets of scans along the precursor *m*/*z* dimension. In general, multiple separation techniques could be combined, such as elution time, ion mobility, precursor isolation *m*/*z,* and, as long as a unique signal corresponding to the chemical constituent of interest can be determined, the methods of the present teachings may be usefully applied.

Below, there are described some interesting cases where additional selectivity could be obtained by varying multiple parameters. In a first case, FAIMS is employed. FAIMS devices are frequently incorporated into mass spectrometer instruments between an ion source and a vacuum chamber of a mass spectrometer, where they are used to provide an initial partial separation or pre-filtering of ions according to the ions' differential ion mobility. Schweppe and coworkers have already demonstrated that FAIMS pre-separation of ions in combination with MS2 analysis does reduce, but does not completely eliminate, ratio compression. Methods in accordance with the present teachings may further reduce ratio compression. For example, peptides can be first identified in a discovery experiment, where two (or more) different FAIMS compensation voltages (CV) are used, with enough separation between the two compensation voltages that a peptide identified at one CV has a low probability of being measured at the second CV, or where the relative amounts of the peptide at the two CV's at least change. One way to utilize the FAIMS and retention time separation together, in follow-up quantitative experiments, to resolve the pure reporter ion signal that is associated with the peptide of interest would be to use DIA, where the instrument is directed to: (1) acquire MS2 spectra that span a precursor m/z range using FAIMS pre-filtering employing the first CV, and then (2) acquire further MS2 spectra that span the same range again using FAIMS pre-filtering employing the second CV. For a peptide of interest, the *m*/*z* channels that are known to contain fragments are interrogated. Using this technique, it will be possible to identify a unique signal that is correlative with the peptide over the elution and CV space by computing the median normalized intensity value of these *m*/*z* channels. Intensities in *m*/*z* channels that do not correlate with this unique pattern would be assigned to the Unknown column in Equation 1.

According to some embodiments of the present teachings, a Trapped Ion Mobility (TIMS) apparatus, such as is taught in U.S. Pat. No. 7,838,826 U.S. Pat. No. 9,683,964 may be employed as a pre-filtering device. One embodiment of a TIMS apparatus comprises an elongated ion tunnel through which ions pass. The boundary of the tunnel is defined by a plurality of parallel segmented diaphragms having electrodes thereon. The ion tunnel is divided into an accumulation tunnel portion and a mobility scan/separation tunnel portion. The apparatus includes two voltage supply units for the two tunnel units. The apparatus also comprises an entrance ion funnel at an inlet end and an exit ion funnel at an outlet end. Other versions of mobility spectrometer apparatuses as taught in U.S. Pat. No. 7,838,826 lack the accumulation tunnel section and, instead, utilize the entrance funnel portion for ion accumulation. The separation of ion species within the TIMS device is based upon a gas flow in the cylindrical separation tunnel which drives the ions from an ion source in an accumulation phase against a counter-acting electric DC field barrier while, simultaneously, the ions are radially confined by a quadrupolar RF field.

The counteracting forces upon ions that are generated by gas flow and electric field may be manipulated to cause different species of ions to be separated from one another as they move through a TIMS device at different rates. Moreover, the rate at which the separated ion species are released from a TIMS device to a downstream mass analyzer may be controlled by controlling the gas flow and electric field parameters. Therefore, in the context of the present disclosure, control of these TIMS operational parameters may be used to control the form of signal versus time profiles that are recorded by the mass analyzer and that are used purposes of determining correlations between peptide and reporter ion signals.

The third, and final case is another DIA case, described here without FAIMS, but which could be combined with FAIMS as above in a straight-forward way. In this variation, one can use a series of DIA mass spectral analyses comprising overlapping m/z windows to achieve higher selectivity than that afforded by the nominal isolation width. For example, one could use 6 Th isolation windows, and acquire MS2 scans that span a precursor m/z range in 3 mini-cycles, where the start of each mini-cycle is shifted by 2 Th from the previous mini-cycle. So the first cycle would, for example, isolate ions in *m*/*z* windows centered *m*/*z* values {396,402, ... 900}, the second cycle would isolate ions in windows centered at *m*/*z* values {398,404, ...,902}, and the third cycle would isolate ions in windows centered at *m*/*z* {400,406, ...,904}. Any compound, for example one at m/z 400, would be characterized 3 times over the 3 mini-cycles: once with isolation @ m/z 398, again @ m/z 400, and finally @ m/z 402. The probability of an interference having the same signal pattern as the bona fide signal from the peptide of interest is lower using these signals than it would be if only the *m*/*z* 400 scan were acquired, for example. One way to combine this technique with FAIMS would be to utilize a different CV for each of the mini-cycles. It is further worth noting that both the above "DIA" examples described above could easily be adapted to a DDA method that utilizes a real time search filter. If the initial MS2 DDA scan were to produce a spectral match to a peptide of interest, then the same precursor could be re-interrogated using FAIMS CV values or MS2 isolation windows that subtly varied from the original MS2 scan.

The reporter-ion ratio distortion methods of the present teachings have been described, in the above discussion, as depending on "finding" a unique signal for a peptide of interest as experimental parameters are varied. According to an alternative strategy, variations of the presently-taught methods may include *a priori* estimations of how the signal of the peptide of interest signal should vary across various of the parameter spaces. For example, IMS, FAIMS, and LC peaks can be measured or calculated for the components in a sample, and the K or U columns in Equation 1 could therefore be constructed based on this knowledge, and not on a post-acquisition analysis of the signals. Such strategies may improve results, especially when the number of components is large.

FIG. 5 is a flow diagram of an exemplary method **80** in accordance with the present teachings. The method **80** pertains to correction of reporter-ion abundance ratios between pairs of reporter ions that are detected during tandem mass analysis (MS2), where the reporter ions are liberated by the MS2 fragmentation and are derived from labeled peptides comprising a plurality of different isobaric tags, where each isobaric tag comprises: (a) a respective reporter-ion moiety that comprises a respective predetermined reporter-ion formula weight, and (b) a respective mass-balancing moiety that comprises a respective predetermined mass-balancing formula weight, whereby all isobaric labels comprise identical masses.

The step **81 of** the method **80** is the step of liberating the plurality of reporter-ion moieties by fragmentation of one or more of the labeled peptides during a time period. For example, the time period may be a time period during which elution of a specific peptide of interest is expected to occur. In some other examples, the time period may be one of several scheduled time periods associated with a Data-Independent Analysis (DIA) experiment. In some other cases, the time period may be a time period during which an ion species that has been previously at least partially separated from other ion species (e.g., by an ion mobility device) is being transferred to and analyzed by a mass analyzer. Alternatively, the time period may be defined in some different manner.

In step 82, which may occur essentially simultaneously with step 81, a record is made of the time variation of the raw mass spectrometric signals generated by mass spectrometric detection of the signals at the plurality of reporter-ion m/z channels. In step 83, which may occur essentially simultaneously with the steps **81** and 82, a record is made of the time variation of either a mass spectrometric signal or, alternatively, an instrumental parameter or other related variable, that is identified as pertaining to the detection of one or more peptides of interest.

In step 84 of the method 80, identifications are made of: (a) a first set of reporter-ion moieties for which the respective mass spectrometric signals are positively correlated, in accordance with an above-threshold correlation coefficient, with the variation of the identified mass spectrometric signals, parameters or variables and (b) a second set of reporter-ion moieties that are not so correlated. In step 85, each reporter-ion moiety of the first set (i.e., correlated with the peptide-related signal as identified in step 84) is decomposed, based on the correlation, into a first portion that is attributable to the peptide of interest and a second portion that is not attributable to the peptide of interest. For example, the portion of the signal that is found to be not attributable to the peptide of interest may be the result of co-elution, during the time period, of another labeled compound other than the peptide of interest.

In step 86 of the method 80, an adjusted mass spectrometric signal is set or defined, for each reporter-ion moiety of the first set, as identified in step 84, as the portion of the raw reporter-ion signal that is attributable to the peptide of interest. Finally, in step 87, corrected reporter-ion ratios are calculated that are based on the adjusted mass spectrometric signals, as set in step 86. The steps of the method 80 may, in some instances, be performed using the raw mass spectrometric signal traces (e.g., traces **301, 303, 305, 307, 309, 311** of FIG. 3, as well as any traces relating to peptide precursor ion or fragment ion variation) that are measured versus time or, possibly versus some other parameter or variable. However, in many instances, it is preferable to integrate either the raw signals or the final results over time the time variable.

FIG. 6 is an illustration of a mass spectrometer system in accordance with the present teachings that includes a computer or other electronic processor. The system 90 illustrated in FIG. 6 may comprise one or more of: (a) a sample ion source 91 (i.e., a source of sample ions, generally but not exclusively cations); a reagent ion source 94 (generally, a source of anions or cations suitable for initiating fragmentation of selected sample ions or fragments by, for example, electron transfer dissociation); (b) an ion manipulation module **92** comprising one or more ion traps, ion fragmentation or reaction cells, ion mobility separation devices, etc. wherein the ion manipulation module is fluidically coupled to the sample ion source **91** and, optionally, to the reagent ion source so as to receive sample ions and optional reagent ions, respectively; and (c) a mass analyzer **93** fluidically coupled to the ion manipulation module **92** so as to receive sample ions, precursor ions, fragment ions or other product ions for analysis therein. One or more power supplies **51** are electrically coupled to and provide electrical voltages to the sample ion source, the ion traps or reaction cells of the ion manipulation module 92,and the mass analyzer. Provision of voltages or other electrical signals, of appropriate magnitudes and at appropriate times, to electrodes of the ion source, the ion traps or reaction cells of the ion manipulation module and the mass analyzer (including a detector of the mass analyzer) are the means by which the generation and flow of ions through the mass spectrometer system is controlled and, ultimately, the means by which mass analyses are executed.

The system **90** may further comprise a computer or other electronic processor or controller **95** that is electronically coupled to the sample ion source 91, the ion manipulation module 92, the mass analyzer **93** and the reagent ion source 94 so as to provide programmatic control instruction to and receive operational data (feedback) from those devices. The system **90** may further comprise a tangibly-embodied computer-readable medium comprising an input database **96** and a tangibly-embodied computer-writeable medium comprising an output database 96. These tangibly-embodied computer-readable and writeable media, which may, in fact, be the same tangibly-embodied medium may be embodied as or on a magnetic disk drive, a floppy disk, a magnetic tape, an optical disk such as a CD or DVD, a memory card, USB flash memory, etc. The tangibly-embodied computer-readable medium having the input database **96** (or another tangibly-embodied computer-readable medium) may also comprise computer or processor instructions for carrying out instrument control steps or other method steps in accordance with the present teachings. Optionally, the computer/processor **95** may further be electronically coupled to one or more other output devices 97, 99, such as display screens, printers, information storage devices, etc. and/or one or more other input devices 98, such as keyboards, internet connections, etc.

Dashed connecting lines in FIG. 6 represent connection pathways that carry electronic signals and the flow of electronic signal information. Arrows on the various connecting lines represent possible directions of flow of information or, in the case of solid lines, ion propagation direction. The connecting lines between the components **91, 92** and **93** implicitly include any necessary ion transfer optics or ion optical assemblies, etc. These hardware components, themselves, may include any necessary additional power supplies, housings, vacuum lines, etc., even though such ancillary components may not be explicitly shown in the accompanying drawings, for reasons of clarity.

In the system **90** (FIG. 6), the electronic connections between the computer/processor **95** and the sample ion source 91, the reagent ion source 94, the mass analyzer 93, and the ion trap(s) or reaction cells **92** provide a pathway for operational commands to be sent from the computer/processor **95** to these other components. Such commands may include commands for starting and/or stopping one or more processes of ion generation, ion transfer, or ion fragmentation steps according to the methods taught herein and will generally include specific commands to apply (or stop applying) certain voltages to various electrodes or ion optics of the ion sources, ion traps, ion reaction cells, ion mobility separation devices (if any) and mass analyzer. These control lines may also transfer information from various sensors or detectors back to the computer/processor **95.** Information sent from the mass analyzer **93** to the computer/processor **95** will include the raw experimental data, relating to *m*/*z* ratios of detected ions and the magnitude of signal strength associated with each ion, for example, the raw data of detected intensities of reporter ions shown in FIG. 3 but also including, in many case of mass spectral measurements conducted in accordance with the present teachings, such as MS1 and possibly MS2 mass spectral data of peptides. Other information sent to the computer/processor **95** from the mass analyzer **92,** the sample ion source **91,** the reagent ion source **94** and/or the ion trap(s) or reaction cell(s) **92** may include monitor or feedback signals from sensors of these components (for instance, temperature sensors, pressure sensors, ion filling level sensors, etc.) which relate to operational performance.

As illustrated in FIG. 6, the computer/processor **95** may be in electronic communication with a tangibly-embodied computer-readable medium having at least one input database **96** which contains, at least, information used to conduct mass spectral library searches, if applicable. The same tangibly-embodied computer-readable medium may also comprise computer-readable instructions used to conduct various instrumental control and data processing steps in accordance with the present teachings, as described above. The computer/processor **95** may also be in electronic communication with a tangibly-embodied computer-readable medium having at least one output database **97** which contains, at least, results of data collecting and processing steps as taught herein, such as the results of corrected reporter ion ratios, as shown in FIGS 4A-4F, as well as the experimental and data processing parameters employed to calculate such results. Other input devices **98** and output devices **99** may be employed to receive information from or send information to a user, the internet, a local network, a printer, a data display device, etc.

Still with reference to FIG. 6, it is noted that the computer/processor **95** may optionally be electronically connected -- either through the Internet or as part of an interconnected intranet other private network of computers, such as a local area network -- using one or more data-link devices **53** to one more other processors **55** which may share some or all of the calculation load. On the other hand, a user, such as an analytical chemist or instrumental technician, may have a need or desire to have access to and to be able to store results of data processing steps in "real-time" -- that is, simultaneously with data acquisition or shortly after data acquisition. For relatively simple experiments for systems having access to a relatively fast computer or processor, such real time accessing, display or storage of calculation results may be possible utilizing just the computer/processor **95** shown in FIG. 6. However, whereas spectral acquisition time is on the order of only seconds or less, calculations may require up to several minutes of processor time if a sample comprises one or more complex polypeptides. Under such circumstances, a single processor may be insufficient to keep up with the calculation load. However, networked, parallel versions of search programs are known which are designed to run on a cluster of computers, for instance the program called SEQUEST-PVM (Sadygov, R. G.; Eng, J; Durr, E; Saraf, A.; McDonald, H.; MacCoss, M. J.; Yates, J. R.; "Code developments to improve the efficiency of automated MS/MS spectra interpretation"; Jour. Proteome Research, 2002, 1, pp. 211-215). In some instances, off-loading of some or the entire calculation burden to the one more other processors **55** may enable results to be obtained sufficiently quickly to enable real-time provision of calculation results. In such instances, raw or intermediate data may be sent to the one more other processors **55** at the same time that the computer/processor **95** continues to perform instrument control and monitoring and raw data acquisition functions. The results of the calculations performed by the one more other processors **55** may be subsequently sent to the computer/processor **95** for display or output to a user or for storage in the output database **97.**

The discussion included in this application is intended to serve as a basic description. The present invention is not intended to be limited in scope by the specific embodiments described herein, which are intended as single illustrations of individual aspects of the invention. The invention is set out in the appended set of claims.

## Claims

1. A method for correcting abundance ratios between reporter ions detected during tandem mass analysis (MS2) of a sample that comprises a plurality of peptides that are labeled with a plurality of different isobaric tags, each isobaric tag comprising a respective reporter-ion moiety that comprises a respective predetermined reporter-ion formula weight and a respective mass-balancing moiety that comprises a respective predetermined mass-balancing formula weight, the method comprising:
during a time period, measuring, for each reporter-ion moiety liberated by the tandem mass analysis, the variation, with time, of a respective mass spectrometric signal derived from said respective reporter-ion moiety;
identifying a first set of reporter-ion moieties for which the respective mass spectrometric signal is positively correlated, with an above-threshold correlation coefficient, with the time variation, during the time period, of one or more other mass spectrometric signals, parameters or variables that pertain to the detection of a peptide of interest and identifying a second set of reporter-ion moieties for which the respective mass spectrometric signal is not positively correlated with the one or more other mass spectral signals or variables;
based on the correlations, for each identified reporter-ion moiety of the first set, decomposing the respective measured mass spectrometric signal into a first portion that is attributable to the peptide of interest and a second portion that is not attributable to the peptide of interest;
for each identified reporter-ion moiety, setting a respective adjusted mass spectrometric signal as being the respective portion of the mass spectrometric signal that is attributable to the peptide of interest; and
calculating corrected reporter-ion ratios based on the adjusted mass spectrometric signals.

2. A method as recited in claim 1, wherein the decomposing of each measured mass spectrometric signal into a respective first portion that is attributable to the peptide, and a second portion that is not attributable to the peptide comprises calculating the first and second portions using a non-negative least squares analysis.

3. A method as recited in claim 1, wherein the time variation of the one or more other mass spectrometric signals, parameters or variables that pertain to the detection of one or more peptides of interest are determined from tabulated values of peptide elution times and/or elution profiles.

4. A method as recited in claim 1, wherein the time variation of the one or more other mass spectrometric signals, parameters or variables that pertain to the detection of one or more peptides of interest are determined by measuring the variation of the one or more other mass spectrometric signals or variables during the time period.

5. A method as recited in claim 4, wherein the one or more other mass spectrometric signals, parameters or variables that are measured during the time period comprise mass spectra of characteristic peptide fragment ions.

6. A method as recited in claim 1, wherein the beginning of the time period is triggered by a time delay relative to the beginning of an experiment.

7. A method as recited in claim 1, wherein the tandem mass analysis of the sample comprises a data-dependent analysis (DDA) experiment, wherein the beginning of the time period is triggered by detection of an *m*/*z* value, within an MS1 mass spectrum, that corresponds to a peptide of interest.

8. A method as recited in claim 1, wherein the tandem mass analysis of the sample comprises a data-dependent analysis (DDA) experiment, wherein the beginning of the time period is triggered by the prior steps of:
detection of a first *m*/*z* value, within an MS1 mass spectrum, that corresponds to a potential peptide of interest;
automatic consultation of a mass spectral library to determine a second *m*/*z* value to be searched for within a subsequent MS2 mass spectrum;
generation of fragment ions by fragmentation of ions comprising the first *m*/*z* value; and
detection of the second *m*/*z* value within an MS2 spectrum of the fragment ions.

9. A method as recited in claim 1, wherein the tandem mass analysis of the sample comprises a data-independent analysis (DIA) experiment, wherein the time period is one of a plurality of scheduled overlapping time periods.

10. A method as recited in claim 1, wherein the beginning of the time period is triggered by elapse of an ion mobility drift time subsequent to the introduction of a stream of peptide ions labeled with the plurality of different isobaric tags into an ion mobility separation device that receives the ions from an ion source and that delivers ion mobility separated ions to a mass spectrometer that performs the tandem mass analysis.

11. A method as recited in claim 1, wherein the variation, with time, of the mass spectrometric signal derived from each respective reporter-ion moiety and time variation of one or more other mass spectrometric signals or variables is at least partially caused by time variation of a compensation voltage (CV) applied to a Field Asymmetric Ion Mobility Separation (FAIMS) device that that receives ions from an ion source and that delivers ion mobility separated ions to a mass spectrometer that performs the tandem mass analysis.

12. A method as recited in claim 1, wherein the variation, with time, of the mass spectrometric signal derived from each respective reporter-ion moiety and time variation of one or more other mass spectrometric signals or variables is at least partially caused by time variation of one or more operational parameters of a Trapped Ion Mobility Separation (TIMS) device that receives ions from an ion source and that delivers ion mobility separated ions to a mass spectrometer that performs the tandem mass analysis.

13. A mass spectrometer system comprising:
an ion source that generated first-generation ions from a sample;
an ion trap or reaction cell configured to receive first-generation ions from the ion source;
a mass analyzer configured to receive a portion of the first-generation ions or fragment ions generated by fragmentation of a portion of the first-generation ions from the ion trap or reaction cell;
one or more electrical power supplies electrically coupled to the ion source, the ion trap or reaction cell and the mass analyzer; and
a computer processor electrically coupled to the ion source, the ion trap or reaction cell and the mass analyzer and comprising tangibly-embodied non-transitory computer instructions that are operable to cause the one or more power supplies to provide voltages or electrical signals to the ion source, the ion trap or reaction cell and the mass analyzer that cause the mass spectrometer system to execute the method of claim 1.

14. A mass spectrometer system as recited in claim 13, further comprising:
a computer-readable medium that comprises an input database and that is electrically coupled to the computer processor,
wherein the tangibly-embodied non-transitory computer instructions are further operable to receive information from the input database and to cause the one or more power supplies to provide voltages or electrical signals to the ion source, the ion trap or reaction cell and the mass analyzer that cause the mass spectrometer system to execute the method of claim 8.

15. A mass spectrometer system as recited in claim 13, further comprising:
a Field Asymmetric Ion Mobility Separation (FAIMS) device fluidically coupled between the ion source and the ion trap or reaction cell,
wherein the tangibly-embodied non-transitory computer instructions are further operable to provide voltages or electrical signals to the ion source, the ion trap or reaction cell, the FAIMS device and the mass analyzer that cause the mass spectrometer system to execute the method of claim 11.

16. A mass spectrometer system as recited in claim 13, further comprising
a Trapped Ion Mobility Separation (TIMS) device fluidically coupled between the ion source and the ion trap or reaction cell,
wherein the tangibly-embodied non-transitory computer instructions are further operable to provide voltages or electrical signals to the ion source, the ion trap or reaction cell, the TIMS device and the mass analyzer that cause the mass spectrometer system to execute the method of claim 12.

## Patentansprüche

1. Verfahren zur Korrektur von Häufigkeitsverhältnissen zwischen Reporterionen, die während einer Tandem-Massenanalyse (MS2) einer Probe nachgewiesen werden, die eine Vielzahl von Peptiden umfasst, die mit einer Vielzahl von verschiedenen isobaren Markierungen markiert sind, wobei jede isobare Markierung einen jeweiligen Reporterionenrest umfasst, der eine jeweilige vorbestimmte Reporterionen-Formelmasse umfasst, und einen jeweiligen Massenausgleichsrest, der eine jeweilige vorbestimmte Massenausgleichs-Formelmasse umfasst, das Verfahren umfassend:
während einer Zeitspanne, Messen, für jeden durch die Tandem-Massenanalyse freigesetzten Reporterionenrest, der zeitlichen Veränderung eines jeweiligen massenspektrometrischen Signals, das von dem jeweiligen Reporterionenrest abgeleitet ist;
Identifizieren eines ersten Satzes von Reporterionenresten, für die das jeweilige massenspektrometrische Signal mit einem über einem Schwellenwert liegenden Korrelationskoeffizienten positiv mit der zeitlichen Veränderung eines oder mehrerer anderer massenspektrometrischer Signale, Parameter oder Variablen während des Zeitraums korreliert ist, die den Nachweis eines Peptids von Interesse betreffen, und Identifizieren eines zweiten Satzes von Reporterionenresten, für die das jeweilige massenspektrometrische Signal nicht mit dem einen oder den mehreren anderen massenspektralen Signalen oder Variablen positiv korreliert ist;
basierend auf den Korrelationen, für jeden identifizierten Reporterionenrest des ersten Satzes, Zerlegen des jeweiligen gemessenen massenspektrometrischen Signals in einen ersten Teil, der dem Peptid von Interesse zuzuordnen ist, und einen zweiten Teil, der nicht dem Peptid von Interesse zuzuordnen ist;
für jeden identifizierten Reporterionenrest, Festlegen eines jeweiligen angepassten massenspektrometrischen Signals als den jeweiligen Teil des massenspektrometrischen Signals, der auf das Peptid von Interesse zurückzuführen ist; und
Berechnen korrigierter Reporterionenverhältnisse basierend auf den angepassten massenspektrometrischen Signalen.

2. Verfahren nach Anspruch 1, wobei das Zerlegen jedes gemessenen massenspektrometrischen Signals in einen jeweiligen ersten Teil, der dem Peptid zuzuordnen ist, und einen zweiten Teil, der dem Peptid nicht zuzuordnen ist, Berechnen des ersten und zweiten Teils unter Verwendung einer nicht-negativen Analyse der kleinsten Quadrate umfasst.

3. Verfahren nach Anspruch 1, wobei die zeitliche Veränderung des einen oder der mehreren anderen massenspektrometrischen Signale, Parameter oder Variablen, die sich auf den Nachweis eines oder mehrerer Peptide von Interesse beziehen, aus tabellierten Werten von Peptidelutionszeiten und/oder - elutionsprofilen bestimmt wird.

4. Verfahren nach Anspruch 1, wobei die zeitliche Veränderung des einen oder der mehreren anderen massenspektrometrischen Signale, Parameter oder Variablen, die sich auf den Nachweis eines oder mehrerer Peptide von Interesse beziehen, durch Messen der Veränderung des einen oder der mehreren anderen massenspektrometrischen Signale oder Variablen während der Zeitspanne bestimmt wird.

5. Verfahren nach Anspruch 4, wobei das eine oder die mehreren anderen massenspektrometrischen Signale, Parameter oder Variablen, die während der Zeitspanne gemessen werden, Massenspektren von charakteristischen Peptidfragmentionen umfassen.

6. Verfahren nach Anspruch 1, wobei der Beginn der Zeitspanne durch eine Zeitverzögerung relativ zum Beginn eines Experiments ausgelöst wird.

7. Verfahren nach Anspruch 1, wobei die Tandem-Massenanalyse der Probe ein Experiment zur datenabhängigen Analyse (DDA) umfasst, wobei der Beginn der Zeitspanne durch Nachweis eines *m*/*z*-Werts innerhalb eines MS1-Massenspektrums ausgelöst wird, der einem Peptid von Interesse entspricht.

8. Verfahren nach Anspruch 1, wobei die Tandem-Massenanalyse der Probe ein Experiment zur datenabhängigen Analyse (DDA) umfasst, wobei der Beginn der Zeitspanne durch die folgenden vorherigen Schritte ausgelöst wird:
Nachweis eines ersten *m*/*z*-Werts innerhalb eines MS1-Massenspektrums, der einem potenziellen Peptid von Interesse entspricht;
automatische Konsultation einer Massenspektrenbibliothek, um einen zweiten *m*/*z*-Wert zu bestimmen, nach dem innerhalb eines nachfolgenden MS2-Massenspektrums gesucht werden soll;
Erzeugen von Fragmentionen durch Fragmentierung von Ionen, die den ersten *m*/*z*-Wert umfassen; und
Nachweis des zweiten *m*/*z*-Werts innerhalb eines MS2-Spektrums der Fragmentionen.

9. Verfahren nach Anspruch 1, wobei die Tandem-Massenanalyse der Probe ein Experiment zur datenunabhängigen Analyse (DIA) umfasst, wobei die Zeitspanne eine einer Vielzahl von geplanten überlappenden Zeitspannen ist.

10. Verfahren nach Anspruch 1, wobei der Beginn der Zeitspanne durch den Ablauf einer Ionenmobilitätsdriftzeit im Anschluss an die Einleitung eines Stroms von Peptidionen, die mit der Vielzahl von verschiedenen isobaren Markierungen markiert sind, in eine Ionenmobilitäts-Vereinzelungseinrichtung ausgelöst wird, die die Ionen von einer Ionenquelle empfängt und die nach Ionenmobilität getrennten Ionen an ein Massenspektrometer liefert, das die Tandem-Massenanalyse durchführt.

11. Verfahren nach Anspruch 1, wobei die zeitliche Veränderung des massenspektrometrischen Signals, das von jedem jeweiligen Reporterionenrest abgeleitet ist, und die zeitliche Veränderung eines oder mehrerer anderer massenspektrometrischer Signale oder Variablen mindestens teilweise durch eine zeitliche Veränderung einer Kompensationsspannung (CV) verursacht wird, die an eine Vorrichtung zur feldasymmetrischen Ionenmobilitätsseparation (FAIMS) angelegt wird, die Ionen von einer Ionenquelle empfängt und die ionenmobilitätsgetrennte Ionen an ein Massenspektrometer liefert, das die Tandem-Massenanalyse durchführt.

12. Verfahren nach Anspruch 1, wobei die zeitliche Veränderung des massenspektrometrischen Signals, das von jedem jeweiligen Reporterionenrest abgeleitet ist, und die zeitliche Veränderung eines oder mehrerer anderer massenspektrometrischer Signale oder Variablen mindestens teilweise durch die zeitliche Veränderung eines oder mehrerer Betriebsparameter einer Trapped-Ion-Mobility-Separation-Vorrichtung (TIMS-Vorrichtung) verursacht wird, die Ionen von einer Ionenquelle empfängt und die ionenmobilitätsgetrennte Ionen an ein Massenspektrometer liefert, das die Tandem-Massenanalyse durchführt.

13. Massenspektrometersystem, umfassend:
eine Ionenquelle, die Ionen der ersten Generation aus einer Probe erzeugt hat;
eine Ionenfalle oder Reaktionszelle, die konfiguriert ist, um Ionen der ersten Generation von der Ionenquelle zu empfangen;
einen Massenanalysator, der konfiguriert ist, um einen Teil der Ionen der ersten Generation oder Fragmentionen, die durch Fragmentierung eines Teils der Ionen der ersten Generation erzeugt werden, aus der Ionenfalle oder Reaktionszelle zu empfangen;
eine oder mehrere elektrische Stromversorgungen, die elektrisch an die Ionenquelle, die Ionenfalle oder Reaktionszelle und den Massenanalysator gekoppelt sind; und
einen Computerprozessor, der elektrisch an die Ionenquelle, die Ionenfalle oder Reaktionszelle und den Massenanalysator gekoppelt ist und physisch verkörperte nichtflüchtige Computeranweisungen umfasst, die betreibbar sind, um die eine oder die mehreren Stromversorgungen dazu veranlassen, Spannungen oder elektrische Signale an die Ionenquelle, die Ionenfalle oder Reaktionszelle und den Massenanalysator bereitzustellen, die das Massenspektrometersystem dazu veranlassen, das Verfahren nach Anspruch 1 auszuführen.

14. Massenspektrometersystem nach Anspruch 13, ferner umfassend:
ein computerlesbares Medium, das eine Eingabedatenbank umfasst und das elektrisch an den Computerprozessor gekoppelt ist,
wobei die physisch verkörperten, nichtflüchtigen Computeranweisungen ferner betreibbar sind, um Informationen aus der Eingabedatenbank zu empfangen und zu veranlassen, dass die eine oder die mehreren Stromversorgungen Spannungen oder elektrische Signale an die Ionenquelle, die Ionenfalle oder Reaktionszelle und den Massenanalysator bereitstellen, die das Massenspektrometersystem dazu veranlassen, das Verfahren nach Anspruch 8 auszuführen.

15. Massenspektrometersystem nach Anspruch 13, ferner umfassend:
eine Vorrichtung zur feldasymmetrischen Ionenmobilitätsseparation (FAIMS), die fluidisch zwischen der Ionenquelle und der Ionenfalle oder Reaktionszelle gekoppelt ist,
wobei die physisch verkörperten nichtflüchtigen Computeranweisungen ferner betreibbar sind, um Spannungen oder elektrische Signale an die Ionenquelle, die Ionenfalle oder Reaktionszelle, die FAIMS-Vorrichtung und den Massenanalysator bereitzustellen, die das Massenspektrometersystem dazu veranlassen, das Verfahren nach Anspruch 11 auszuführen.

16. Massenspektrometersystem nach Anspruch 13, ferner umfassend:
eine Trapped-Ion-Mobility-Separation-Vorrichtung (TIMS-Vorrichtung), die fluidisch zwischen der Ionenquelle und der Ionenfalle oder Reaktionszelle gekoppelt ist,
wobei die physisch verkörperten nichtflüchtigen Computeranweisungen ferner betreibbar sind, um Spannungen oder elektrische Signale an die Ionenquelle, die Ionenfalle oder Reaktionszelle, die TIMS-Vorrichtung und den Massenanalysator bereitzustellen, die das Massenspektrometersystem dazu veranlassen, das Verfahren nach Anspruch 12 auszuführen.

## Revendications

1. Procédé de correction de rapports d'abondance entre des ions rapporteurs détectés lors d'une analyse de masse en tandem (MS2) d'un échantillon qui comprend une pluralité de peptides qui sont marqués avec une pluralité de marqueurs isobares différents, chaque marqueur isobare comprenant un fragment d'ion rapporteur respectif qui comprend un poids de formule d'ion rapporteur prédéterminé respectif et un fragment d'équilibrage de masse respectif qui comprend un poids de formule d'équilibrage de masse prédéterminé respectif, le procédé comprenant :
au cours d'un laps de temps, la mesure, pour chaque fragment d'ion rapporteur libéré par l'analyse de masse en tandem, de la variation, dans le temps, d'un signal de spectrométrie de masse respectif dérivé dudit fragment d'ion rapporteur respectif ;
l'identification d'un premier ensemble de fragments d'ions rapporteurs pour lesquels le signal de spectrométrie de masse respectif est corrélé positivement, avec un coefficient de corrélation supérieur à un seuil, à la variation temporelle, pendant le laps de temps, d'un ou plusieurs autres signaux, paramètres ou variables de spectrométrie de masse qui se rapportent à la détection d'un peptide d'intérêt et l'identification d'un second ensemble de fragments d'ions rapporteurs pour lesquels le signal de spectrométrie de masse respectif n'est pas corrélé positivement au ou aux autres signaux ou variables de spectre de masse ;
sur la base des corrélations, pour chaque fragment d'ion rapporteur identifié du premier ensemble, la décomposition du signal de spectrométrie de masse mesuré respectif en une première partie qui est attribuable au peptide d'intérêt et une seconde partie qui n'est pas attribuable au peptide d'intérêt ;
pour chaque fragment d'ion rapporteur identifié, la définition d'un signal de spectrométrie de masse ajusté respectif en tant que partie respective du signal de spectrométrie de masse qui est attribuable au peptide d'intérêt ; et
le calcul de rapports d'ions rapporteurs corrigés sur la base des signaux de spectrométrie de masse ajustés.

2. Procédé selon la revendication 1, dans lequel la décomposition de chaque signal de spectrométrie de masse mesuré en une première partie respective qui est attribuable au peptide, et une seconde partie qui n'est pas attribuable au peptide, comprend le calcul des première et seconde parties à l'aide d'une analyse des moindres carrés non négatifs.

3. Procédé selon la revendication 1, dans lequel la variation temporelle du ou des autres signaux, paramètres ou variables de spectrométrie de masse qui se rapportent à la détection d'un ou plusieurs peptides d'intérêt sont déterminées à partir de valeurs tabulées de temps d'élution et/ou de profils d'élution de peptides.

4. Procédé selon la revendication 1, dans lequel la variation temporelle du ou des autres signaux, paramètres ou variables de spectrométrie de masse qui se rapportent à la détection d'un ou plusieurs peptides d'intérêt est déterminée en mesurant la variation du ou des autres signaux ou variables de spectrométrie de masse pendant le laps de temps.

5. Procédé selon la revendication 4, dans lequel le ou les autres signaux, paramètres ou variables de spectrométrie de masse qui sont mesurés pendant le laps de temps comprennent des spectres de masse d'ions de fragments peptidiques caractéristiques.

6. Procédé selon la revendication 1, dans lequel le début du laps de temps est déclenché par un délai par rapport au début d'une expérience.

7. Procédé selon la revendication 1, dans lequel l'analyse de masse en tandem de l'échantillon comprend une expérience d'analyse dépendante des données (DDA), dans lequel le début du laps de temps est déclenché par la détection d'une valeur *m*/*z*, dans un spectre de masse MS1, qui correspond à un peptide d'intérêt.

8. Procédé selon la revendication 1, dans lequel l'analyse de masse en tandem de l'échantillon comprend une expérience d'analyse dépendante des données (DDA), dans lequel le début du laps de temps est déclenché par les étapes préalables de :
détection d'une première valeur *m*/*z*, dans un spectre de masse MS1, qui correspond à un peptide d'intérêt potentiel ;
consultation automatique d'une banque de spectres de masse pour déterminer une seconde valeur *m*/*z* à rechercher dans un spectre de masse MS2 ultérieur ;
génération d'ions fragments par fragmentation d'ions comprenant la première valeur *m*/*z* ; et
détection de la seconde valeur *m*/*z* dans un spectre MS2 des ions fragments.

9. Procédé selon la revendication 1, dans lequel l'analyse de masse en tandem de l'échantillon comprend une expérience d'analyse indépendante des données (DIA), dans lequel le laps de temps est l'un d'une pluralité de laps de temps programmés se chevauchant.

10. Procédé selon la revendication 1, dans lequel le début du laps de temps est déclenché par l'écoulement d'un temps de dérive de mobilité ionique suite à l'introduction d'un flux d'ions peptidiques marqués avec la pluralité de différents marqueurs isobares dans un dispositif de séparation par mobilité ionique qui reçoit les ions d'une source d'ions et qui délivre des ions séparés par mobilité ionique à un spectromètre de masse qui effectue l'analyse de masse en tandem.

11. Procédé selon la revendication 1, dans lequel la variation, dans le temps, du signal de spectrométrie de masse dérivé de chaque fragment d'ion rapporteur respectif et la variation dans le temps d'un ou plusieurs autres signaux ou variables de spectrométrie de masse est au moins partiellement provoquée par la variation dans le temps d'une tension de compensation (CV) appliquée à un dispositif de séparation par mobilité ionique asymétrique à champ (FAIMS) qui reçoit des ions d'une source d'ions et qui délivre des ions séparés par mobilité ionique à un spectromètre de masse qui effectue l'analyse de masse en tandem.

12. Procédé selon la revendication 1, dans lequel la variation, dans le temps, du signal de spectrométrie de masse dérivé de chaque fragment d'ion rapporteur respectif et la variation dans le temps d'un ou plusieurs autres signaux ou variables de spectrométrie de masse est au moins partiellement provoquée par la variation dans le temps d'un ou plusieurs paramètres de fonctionnement d'un dispositif de séparation par mobilité ionique piégée (TIMS) qui reçoit des ions d'une source d'ions et qui délivre des ions séparés par mobilité ionique à un spectromètre de masse qui effectue l'analyse de masse en tandem.

13. Système de spectromètre de masse comprenant :
une source d'ions qui a généré des ions de première génération à partir d'un échantillon ;
un piège à ions ou une cellule de réaction configuré pour recevoir des ions de première génération à partir de la source d'ions ;
un analyseur de masse configuré pour recevoir une partie des ions de première génération ou des ions fragments générés par fragmentation d'une partie des ions de première génération provenant du piège à ions ou de la cellule de réaction ;
une ou plusieurs alimentations électriques couplées électriquement à la source d'ions, au piège à ions ou à la cellule de réaction et à l'analyseur de masse ; et
un processeur informatique couplé électriquement à la source d'ions, au piège à ions ou à la cellule de réaction et à l'analyseur de masse et comprenant des instructions informatiques non transitoires incorporées de manière tangible qui sont utilisables pour amener la ou les alimentations à fournir des tensions ou des signaux électriques à la source d'ions, au piège à ions ou à la cellule de réaction et à l'analyseur de masse qui amènent le système de spectromètre de masse à exécuter le procédé selon la revendication 1.

14. Système de spectromètre de masse selon la revendication 13, comprenant en outre :
un support lisible par ordinateur qui comprend une base de données d'entrée et qui est couplé électriquement au processeur d'ordinateur,
dans lequel les instructions informatiques non transitoires incorporées de manière tangible sont en outre utilisables pour recevoir des informations à partir de la base de données d'entrée et pour amener la ou les alimentations à fournir des tensions ou des signaux électriques à la source d'ions, au piège à ions ou à la cellule de réaction et à l'analyseur de masse qui amènent le système de spectromètre de masse à exécuter le procédé selon la revendication 8.

15. Système de spectromètre de masse selon la revendication 13, comprenant en outre :
un dispositif de séparation par mobilité ionique asymétrique à champ (FAIMS) raccordé fluidiquement entre la source d'ions et le piège à ions ou la cellule de réaction,
dans lequel les instructions informatiques non transitoires incorporées de manière tangible sont en outre utilisables pour fournir des tensions ou des signaux électriques à la source d'ions, au piège à ions ou à la cellule de réaction, au dispositif FAIMS et à l'analyseur de masse qui amènent le système de spectromètre de masse à exécuter le procédé selon la revendication 11.

16. Système de spectromètre de masse selon la revendication 13, comprenant en outre
un dispositif de séparation par mobilité ionique piégée (TIMS) raccordé fluidiquement entre la source d'ions et le piège à ions ou la cellule de réaction,
dans lequel les instructions informatiques non transitoires incorporées de manière tangible sont en outre utilisables pour fournir des tensions ou des signaux électriques à la source d'ions, au piège à ions ou à la cellule de réaction, au dispositif TIMS et à l'analyseur de masse qui amènent le système de spectromètre de masse à exécuter le procédé selon la revendication 12.
